(19) Europäisches Patentamt
European Patent Office
Office européen des brevets

(11) Numéro de publication: **0 133 110**
**B1**

# (12) FASCICULE DE BREVET EUROPEEN

(45) Date de publication du fascicule du brevet: **17.02.88**

(51) Int. Cl.⁴: **A 61 K 31/19**, A 61 K 31/20, A 61 K 47/00, A 61 K 9/20

(21) Numéro de dépôt: **84401514.9**

(22) Date de dépôt: **18.07.84**

## (54) Nouvelle composition pharmaceutique à base d'acide valproique et son procédé de fabrication.

(30) Priorité: **20.07.83 FR 8312375**
**20.07.83 FR 8312376**

(43) Date de publication de la demande:
**13.02.85 Bulletin 85/7**

(45) Mention de la délivrance du brevet:
**17.02.88 Bulletin 88/7**

(84) Etats contractants désignés:
**AT BE CH DE FR GB IT LI LU NL SE**

(56) Documents cités:
**WO - A - 81/00562**
**US - A - 3 325 361**
**US - A - 4 301 176**

**ROTE LISTE, 1982, Editio Cantor, Aulendorf, DE; No. 14 017 "Convulex", 14 018 "Ergenyl", 14 019 "Leptilan", 14 020 "Mylproin", 14 021 "Orfiril"**
**HAGERS HANDBUCH DER PHARMAZEUTISCHEN PRAXIS, 4e "Neuausgabe", vol. VII-B, Springer-Verlag, Berlin, Heidelberg, New York, 1977, pages 249-251, "Gefällte Kieselsäuren"**

**Le dossier contient des informations techniques présentées postérieurement au dépôt de la demande et ne figurant pas dans le présent fascicule.**

(73) Titulaire: **SANOFI, 40, Avenue George V, F-75008 Paris (FR)**

(72) Inventeur: **Aubert, Daniel, Chemin d'Encrabe, F-31170 Plaisance Du Touch (FR)**
Inventeur: **Blanc, Francis, Chemin de Soriech, F-34000 Lattes (FR)**
Inventeur: **Desmolin, Henri, 27 rue Fontaine d'Arlac, F-33700 Merignac (FR)**
Inventeur: **Morre, Michel, 11 rue St. Odile, F-31100 Toulouse (FR)**
Inventeur: **Sindely, Lucette, Salignac, F-33240 St. Andre De Cubzac (FR)**

(74) Mandataire: **Bressand, Georges et al, c/o CABINET LAVOIX 2 Place d'Estienne d'Orves, F-75441 Paris Cedex 09 (FR)**

**EP 0 133 110 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'acide dipropylacétique ou acide valproïque (D C I) de formule

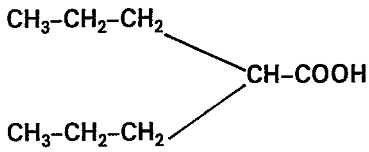

et le valproate de sodium sont utilisés en thérapeutique humaine pour leurs propriétés antiépileptiques. C'est ainsi que le valproate de sodium est administré sous différentes formes galéniques, telles que le soluté buvable ou les comprimés. Il est connu qu'après administration orale, la biodisponibilité sanguine de l'acide valproïque est proche de 100% et que la concentration maximale, qui est atteinte rapidement, peut provoquer des effets secondaires.

D'autre part, la fabrication des comprimés précédemment cités présente différents inconvénients qu'il apparaît nécessaire de rappeler cidessous:

1. la granulation ne peut se réaliser qu'en présence d'un agent liant et avec un solvant de mouillage;
2. avant compression, il est nécessaire d'ajouter un produit adsorbant et un lubrifiant;
3. le noyau ainsi réalisé ne peut recevoir un éventuel enrobage à délitement gastrique qu'après avoir été lui-même enrobé d'un vernis isolant;
4. le poids terminal du comprimé est relativement important, les excipients représentant près de 40% de poids total dudit comprimé;
5. toutes ces opérations doivent être réalisées en atmosphère déshydratée à 30% d'humidité relative.

La présente invention a pour objet de nouvelles compositions pharmaceutiques administrables par voie orale et contenant un mélange d'acide valproïque et de valproate de sodium.

Elle vise plus particulièrement la préparation de comprimés dont le poids final est nettement inférieur à celui obtenu par des procédés classiques de fabrication galénique.

Selon WO-A-8 100 562 on connaît un dimère stable de l'acide valproïque et de son sel de sodium ou de calcium, contenant une proportion mole à mole (rapporté à l'acide valproïque) de ces constituants, c'est-à-dire 46,5%/53,5% (sel de sodium) ou 47%/53% (sel de calcium). Ce document ne permet cependant pas de résoudre les problèmes liés à la fabrication de comprimés contenant du valproate de sodium hygroscopique.

L'invention a pour objet une composition pharmaceutique par voie orale, présentée sous forme de comprimés, qui est caractérisée en ce qu'elle contient, à titre de principes actifs non combinés de 25 à 35% en poids d'acide valproïque et de 75 à 65% de valproate de sodium.

La demanderesse a également découvert que l'emploi, dans un même comprimé, d'acide valproïque, de valproate de sodium, et d'un excipient à effet retard, présentait l'avantage inattendu d'éviter que la concentration maximale sanguine du principe actif provoque des effets secondaires (dus à l'augmentation importante de la concentration d'acide valproïque libre), tout en maintenant une concentration sanguine utile dans le cadre d'une action retardée; il présente également l'avantage d'améliorer de manière inattendue le procédé de fabrication de ce comprimé et d'éviter ainsi les différents inconvénients galéniques précédemment cités.

L'invention a donc pour objet la fabrication de spécialités pharmaceutiques répondant aux critères précédents.

Il existe en effet sur le marché pharmaceutique français des spécialités pharmaceutiques titrant soit 200 mg, soit 500 mg de valproate de sodium par unité de prise, l'excipient utilisé étant composé de silicate de calcium excipient, de polyvidone excipient, de stéarate de magnésium, de talc, de polyoxyéthylène glycol 400, d'amidon de maïs, d'oxyde de titane, d'oxyde jaune de fer, d'acétophtalate de cellulose et de phtalate diéthylique, le poids terminal étant par exemple de 800 mg pour le comprimé enrobé titrant 500 mg de valproate de sodium, correspondant à 434 mg d'acide valproïque.

Les procédés de fabrication des spécialités à base d'acide valproïque ou d'un de ses sels pharmaceutiques acceptables passent tous par le stade d'une granulation réalisée avec un agent liant, comme le polyvidone excipient, et avec un solvant de mouillage, comme l'alcool isopropylique ou comme l'eau. Au grain ainsi obtenu, sont ajoutés avant compression, un produit absorbant, comme le silicate de calcium excipient et un lubrifiant, comme le stéarate de magnésium; après compression, le noyau ainsi réalisé reçoit une couche isolante composée de polyvidone ou de méthacrylate, puis, éventuellement, un enrobage à délitement entérique. Ainsi, le noyau représente un poids de 562,5 mg et la couche isolante et l'enrobage un poids de 237,5 mg conduisant à un poids final important de 800 mg.

Il est ainsi apparu nécessaire d'améliorer les procédés de fabrication de ces spécialités pharmaceutiques afin d'obtenir un poids final de comprimé inférieur à 800 mg, tout en conservant la même quantité de principe actif exprimée en acide valproïque et les mêmes caractéristiques de biodisponibilité.

L'invention a donc pour objet la fabrication d'une spécialité répondant aux caractéristiques précédentes, les procédés de fabrication étant simplifiés et améliorés.

C'est ainsi que le grain pour compression est formé directement par simple mélange dans les proportions précitées d'acide valproïque et de valproate de sodium, en l'absence de tout liant et de tout solvant de granulation. En effet, l'acide valproïque est ajouté lentement, soit directment, soit par pulvérisation, sur le valproate de sodium;

l'agglomération granuleuse se forme d'elle-même en quelques minutes et les grains ainsi obtenus sont passés sur tamis pour calibrage. Cette opération peut être réalisée dans une atmosphère à 55-60% d'humidité relative, sans risque de reprise d'humidité.

Les qualités de compressibilité de ce grain sont très bonnes, l'acide valproïque jouant de plus le rôle de lubrifiant. Afin d'éviter toute tendance de collage au poinçon, il est apparu nécessaire d'additionner de silice précipitée le grain avant compression.

Un autre avantage de l'invention consiste dans la possibilité éventuelle de pose directe de tout enrobage y compris entérique sur le noyau, ce qui permet de supprimer l'encombrement et les temps de pose de la couche isolante.

Autrement dit, les avantages de l'invention consistent en

- une granulation par simple mélange d'acide valproïque et d'un de ses sels pharmaceutiquement acceptables, sans solvant, donc sans séchage
- une formule simplifiée
- un poids de noyau réduit
- la suppression du travail en atmosphère déshydratée à 30% d'humidité relative
- un noyau moins hygroscopique
- un poids final réduit du comprimé.

Il est décrit ci-après, à titre d'exemple, un procédé réalisé à température ambiante de fabrication détaillé des comprimés de l'invention, les proportions indiquées étant rapportées à 1000 comprimés: 145 g d'acide valproïque - qui se présente sous forme de liquide - sont ajoutés lentement ou pulvérisés sur 333 g de valproate de sodium - qui se présente sous forme de poudre -; après formation rapide d'une agglomération granuleuse, les grains sont passés sur tamis pour obtenir un calibrage. On ajoute 52 g de silice précipitée au grain avant compression et on comprime. Les noyaux ainsi obtenus pèsent unitairement 530 mg, l'acide valproïque représente 30,33% du principe actif et le valproate de sodium 69,67% du principe actif, c'est-à-dire une mole d'acide pour deux moles de sel; exprimé en acide valproïque, chaque comprimé titre 434 mg correspondant à 500 mg de valproate de sodium. Ces comprimés sont vernis par exemple à l'aide d'hydroxypropylméthyl-cellulose (18 mg/comprimé) et de glycérol (7 mg/comprimé) ou de tout autre vernis adéquat.

La fabrication de ces comprimés selon l'invention peut être schématisée comme suit, les quantités indiquées correspondant à la fabrication de 1000 comprimés.

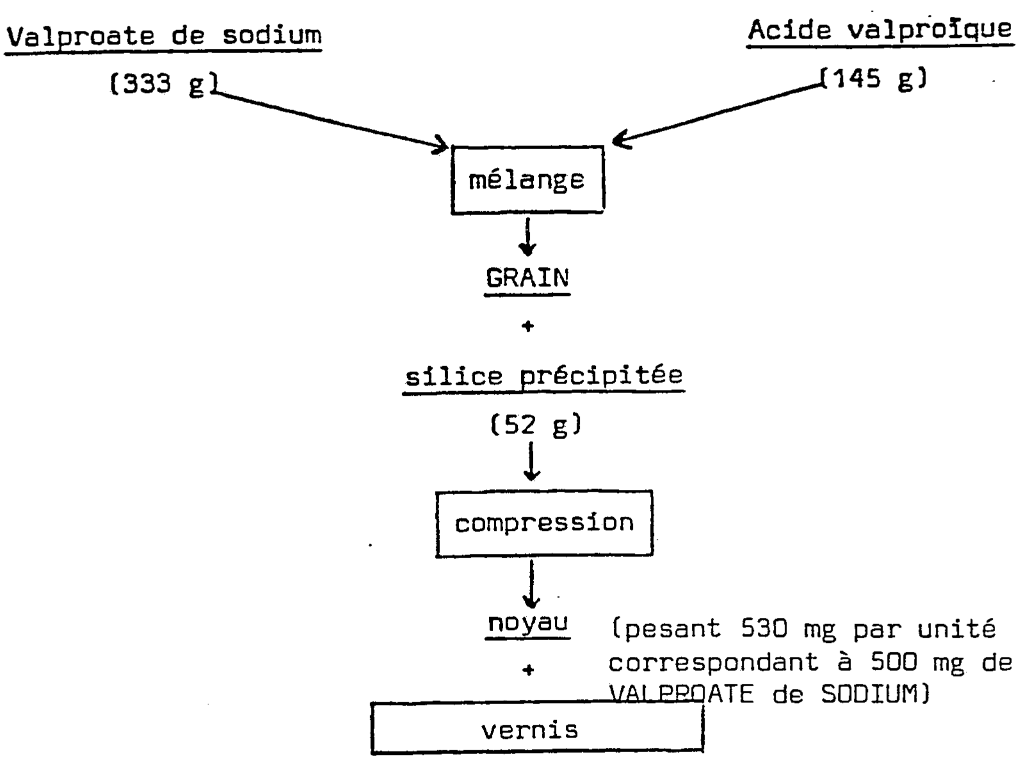


Différents essais ont été réalisés en faisant varier les proportions d'acide valproïque et de valproate de sodium et des résultats convenables ont été obtenus avec des mélanges contenant entre 15 et 48% d'acide valproïque et entre 85 et 52% de valproate de sodium. De même, le comportement à l'humidité du mélange valproate de sodium - acide valproïque a montré qu'après 14 jours de contact à 55 d'humidité relative, le mélange contenant 10% d'acide valproïque et 90% de valproate de sodium a repris 22% d'humidité, celui contenant 20% d'acide valproïque et 80% de valproate de sodium a repris 9% d'humidité; quant au mélange contenant 30% d'acide valproïque et 70% de valproate de sodium, il n'a pratiquement pas repris d'humidité.

Il y a lieu de signaler toutefois qu'il est toujours possible d'ajouter, lors de la fabrication du noyau, différents excipients classiques, en fonction du matériel utilisé et des conditions opératoires. Cette addition, faible en pourcentage, ne modifiera pas les caractéristiques de biodisponibilité du médicament de l'invention qui sont décrites ci-dessous.

Comme il a été signalé plus haut, des essais de biodisponibilité comparée ont été réalisés avec ces comprimés du commerce titrant 500 mg de valproate de sodium et les comprimés de l'invention titrant également 500 mg de valproate de sodium (ou 434 mg d'acide valproïque).

Six volontaires sains, âgés de 24 à 30 ans, ont reçu chacun une prise unique de deux comprimés, correspondant à 1000 mg de valproate de sodium ou à 868 mg d'acide valproïque; l'administration accompagnée d'un verre d'eau, a eu lieu à jeun, le premier repas, pauvre en lipides, survenant environ 4 heures après la prise d'essais. Le produit du commerce et le médicament de l'invention ont été administrés successivement, avec une période de repos d'au moins 8 jours entre deux séquences.

Les dosages sanguins ont été réalisés aux temps suivant: 0, 10 min, 30 min, 45 min, 1 h, 1 h 30, 2 h, 2 h 30, 3 h, 3 h 30, 4 h, 4 h 30, 5 h, 6 h et 8 h, le temps 0 correspondant à la première apparition dans le plasma de traces d'acide valproïque.

Les plasmas ont été séparés par centrifugation à 3000 tours/minute pendant une durée de 5 minutes et congelés jusqu'au moment de l'analyse.

L'acide valproïque a été dosé dans les échantillons par chromatographie gaz-liquide avec détection par ionisation de flamme sur appareil Hewlett-Packard 5710 A, équipé d'un injecteur automatique HP 7672 A.

A 0,5 ml de plasma, il a été ajouté 50 µg de méthyl-1 cyclohexyl carboxylate de sodium (étalon interne) et 0,100 ml d'acide chlorhydrique 0,5 ml de chloroforme par agitation au vortex pendant une minute, puis par centrifugation pendant 15 minutes à 6000 tours/minute. Une gamme d'étalonnage a été préparée de manière identique dans le plasma.

Les concentrations plasmatiques moyennes d'acide valproïque exprimées en µg/ml en fonction du temps sont réunies dans le tableau suivant:

| Produits / Temps | Médicament du Commerce (lot A) | Médicament de l'Invention (lot B) |
|---|---|---|
| 0 | ε | ε |
| 10 min | 45 | 47 |
| 30 min | 90 | 86 |
| 45 min | 97 | 95 |
| 1 h | 89 | 86 |
| 1 h 30 | 87 | 85 |
| 2 h | 85 | 82 |
| 2 h 30 | 74 | 71 |
| 3 h | 70 | 68 |
| 3 h 30 | 65 | 63 |
| 4 h | 62 | 60 |
| 4 h 30 | 57 | 59 |
| 5 h | 55 | 54 |
| 6 h | 51 | 50 |
| 8 h | 45 | 46 |

Il découle des résultats obtenus les points suivants:

1. les biodisponibilités du médicament du commerce et du médicament de l'invention sont sensiblement identiques;

2. la concentration maximale se situe, au temps 45 minutes, à 97 µg/ml pour le lot A et à 95 µg/ml pour le lot B;

3. les aires sous les courbes sont sensiblement identiques pour les deux lots:
- 940±75 pour le lot A
- 949±80 pour le lot B

Ces résultats confirment que la fabrication des comprimés selon le procédé de l'invention ne modifie pas la biodisponibilité du principe actif telle qu'elle existe avec le médicament existant dans le commerce.

Comme il a été signalé ci-dessus, la demanderesse a également découvert que l'emploi, dans un même comprimé, d'acide valproïque associé à l'un de ses sels pharmaceutiquement acceptables, et d'un excipient à effet retard, présentait l'avantage inattendu d'éviter que la concentration maximale sanguine du principe actif provoque des effets secondaires, tout en prolongeant le maintien de cette concentration sanguine, dans le cadre d'une action retardée, à l'intérieur de la zone des concentrations thérapeutiques.

Le grain pour compression est formé directement par simple mélange, dans des proportions convenables d'acide valproïque additionné d'éthylcellulose et d'un de ses sels pharmaceutiquement acceptables additionné ou non d'Eudra-

git®, en l'absence de tout liant et de tout solvant de granulation. Le mélange acide valproïque-éthylcellulose est ajouté lentement sur le mélange de sel d'acide valproïque-Eudragit®; l'agglomération granuleuse se forme d'elle-même en quelques minutes et les grains ainsi obtenus sont passés sur tamis pour calibrage. Cette opération peut être réalisée dans une atmosphère à 55-60% d'humidité relative, sans risque de reprise d'humidité.

Afin d'éviter toute tendance de collage au poinçon, il est apparu nécessaire d'additionner de silice précipitée le grain avant compression.

Il est décrit ci-après, à titre d'exemple non limitatif, un procédé réalisé à température ambiante de fabrication détaillé des comprimés de l'invention, les proportions indiquées étant rapportées à 1000 comprimés: 145 g d'acide valproïque additionnés à 12 g d'éthylcellulose sont ajoutés lentement sur le mélange composé de 333 g de valproate de sodium et de 113 g d'Eudragit®; après formation rapide d'une agglomération granuleuse, les grains sont passés sur tamis pour obtenir un calibrage. On ajoute 52 g de silice précipitée au grain avant compression et on comprime. Les noyaux ainsi obtenus pèsent unitairement 655 mg et sont par exemple vernis avec 18 mg d'hydroxypropylméthylcellulose et 7 mg de glycérol ou tout autre vernis adéquat; on obtient le comprimé C qui correspond à 500 mg de valproate de sodium (soit 434 mg exprimés en acide valproïque).

Une autre variante de l'invention consiste à ne pas ajouter d'Eudragit® aux 333 g de valproate de sodium. Toutes les opérations de fabrication se déroulent de la même manière, le poids unitaire du noyau étant alors de 542 mg et celui du comprimé vernis étant de 567 mg on obtient le comprimé B qui corresspond à 500 mg de valproate de sodium.

D'autres variantes de l'invention consistent à modifier les quantités respectives d'éthylcellulose (de 5 à 125 mg par unité de prise) et d'Eudragit® (de 50 à 150 mg par unité de prise) selon le degré «d'effet retard» souhaité et à se dispenser d'un enrobage entérique sur le noyau.

La fabrication des comprimés selon l'invention peut être schématisée comme suit:

Comprimé C :

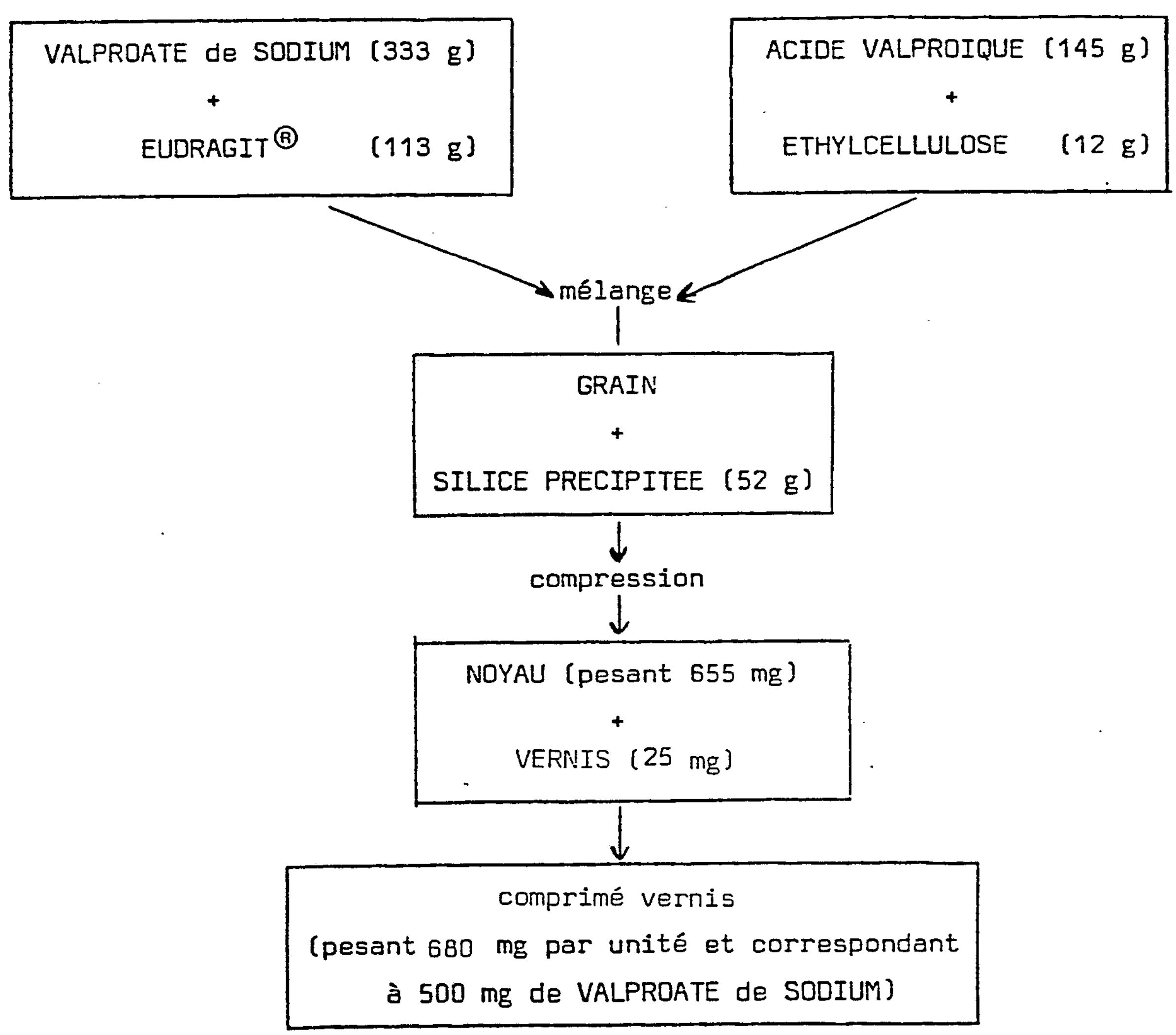

Comprimé D :

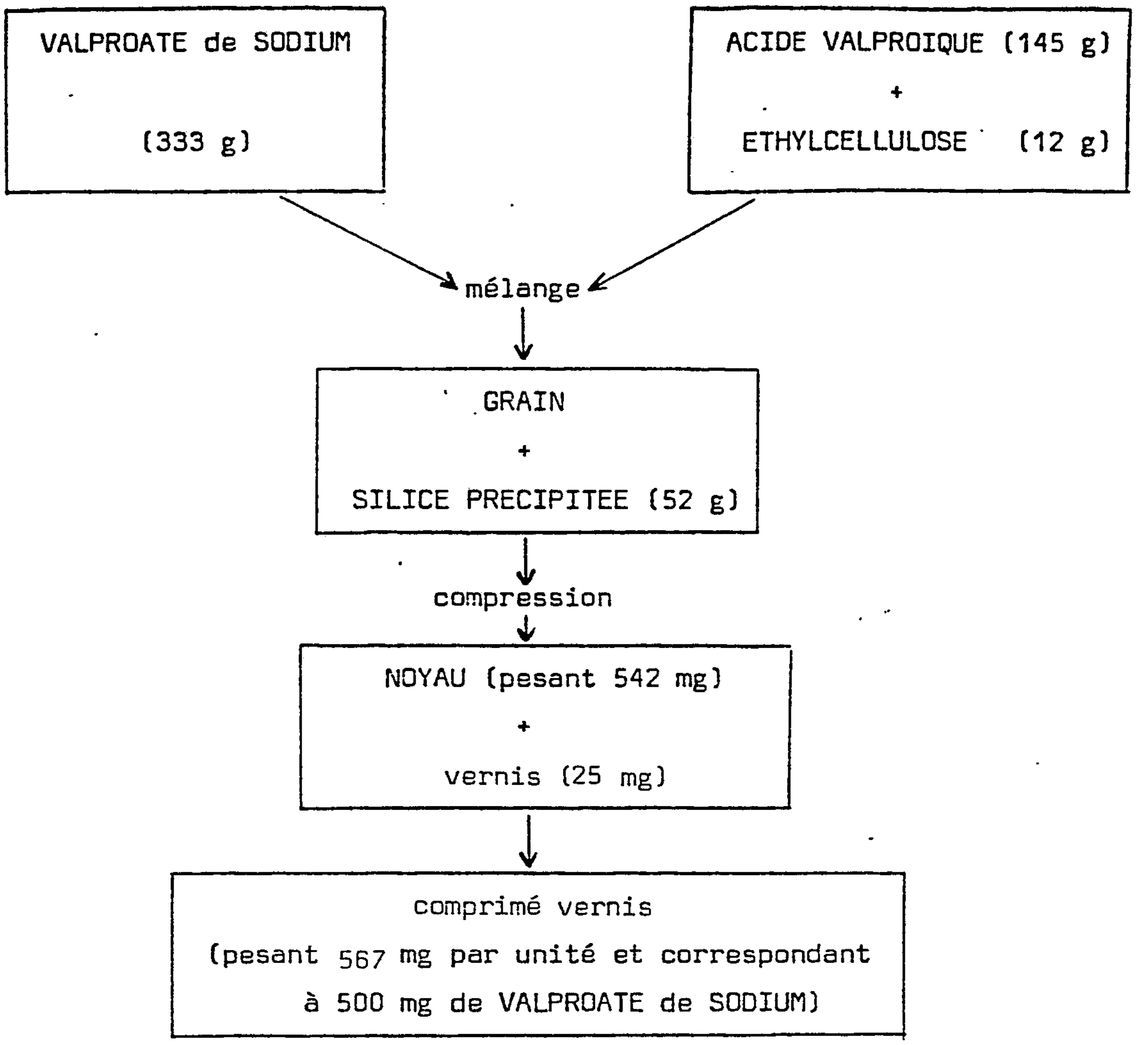


Pour apporter la preuve de l'effet retard du médicament de l'invention, il a été procédé à des essais in vitro de dissolution, et à des essais in vivo de biodisponibilité, le comprimé témoin T ayant été fabriqué de la même manière mais ne contenant ni éthylcellulose ni Eudragit®. La fabrication de ce comprimé T peut être ainsi schématisée:

Comprimé T :

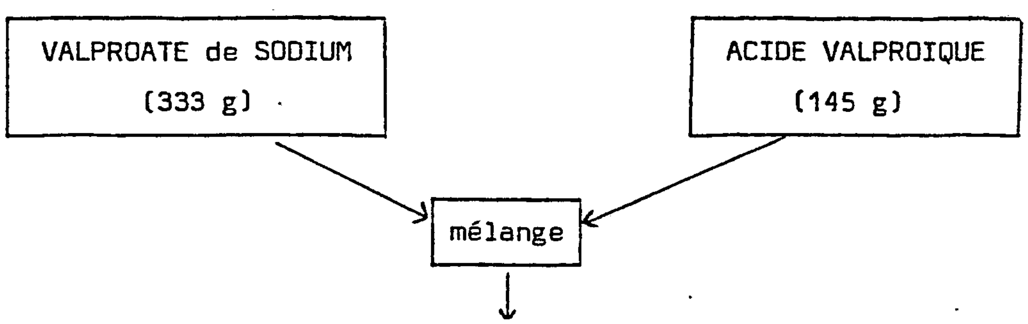

Comprimé T : (suite)

↓

GRAIN
+
SILICE PRECIPITEE (52 g)

↓

compression

↓

NOYAU (pesant 530 mg par unité)
+
vernis (25 mg)

↓

comprimé vernis
(pesant 540 mg par unité et correspondant à 500 mg de VALPROATE de SODIUM)

Essais in vitro (essai de dissolution)

L'essai de dissolution auquel il a été procédé, avait pour but de déterminer la quantité d'acide valproïque libre passé en solution, dans un milieu artificiel, tampon phosphate à pH 6,8, cette quantité de principe actif étant appréciée par dosage dans des échantillons prélevés dans le milieu de dissolution, à plusieurs intervalles de temps prescrits.

L'essai a été réalisé selon la technique décrite dans la note technique no 79 Pro-Pharmacopoe A (Bull. Ordre Pharm, Mars 1980, no 231).

Les résultats obtenus sont consignés dans le tableau suivant. Ils sont exprimés en % par rapport à la quantité d'acide valproïque (434 mg) contenue par unité de prise.

| Produits / Temps | Comprim' | Comprimé C | Comprimé D |
|---|---|---|---|
| 0 | 0 | 0 | 0 |
| 30 min | 60 | 24 | 38 |
| 1 h | 83 | 31 | 49 |
| 1 h 30 | 90 | 35 | 61 |
| 2 h | 95 | 40 | 68 |
| 2 h 30 | 97 | 43 | 73 |
| 3 h | 101 | 45 | 79 |
| 3 h 30 | 102 | 48 | 83 |
| 4 h | 103 | 50 | 87 |
| 4 h 30 | 103 | 53 | 88 |
| 5 h | 103 | 56 | 90 |
| 5 h 30 | 103 | 58 | 93 |
| 6 h | 103 | 60 | 95 |

Essais in vivo (biodisponibilité)

Les essais de biodisponibilité comparée ont été réalisés avec le comprimés T, les comprimés C et les comprimés D, chacun de ces comprimés titrant 500 mg de valproate de sodium.

Six volontaires sains, âgés de 24 à 30 ans, ont

reçu chacun une prise unique de deux comprimés, correspondant à 1000 mg de valproate de sodium ou à 868 mg d'acide valproïque: l'administration accompagnée d'un verre d'eau a eu lieu à jeun, le premier repas, pauvre en lipides, survenant environ 4 heures après la prise d'essai. Les comprimés C, D et T ont été administrés successivement, avec une période de repos d'au moins huit jours entre deux séquences.

Les dosages sanguins ont été réalisés aux temps suivants: 0, 10 min, 30 min, 45 min, 1 h, 1 h 30, 2 h, 2 h 30, 3 h, 3 h 30, 4 h, 4 h 30, 5 h, 6 h et 8 h, le temps 0 correspondant à la première apparition dans le plasma de traces d'acide valproïque.

La méthode d'extraction et de dosage sanguin de l'acide valproïque est identique à celle précédemment décrite.

Les concentrations plasmatiques moyennes d'acide valproïque exprimées en µg/ml en fonction du temps sont réunies dans le tableau suivant:

| Produits / Temps | Comprimé T | Comprimé C | Comprimé D |
|---|---|---|---|
| 0 | ε | ε | ε |
| 10 min | 47 | 15 | 22 |
| 30 min | 86 | 24 | 38 |
| 45 min | 95 | 29 | 38 |
| 1 h | 86 | 35 | 49 |
| 1 h 30 | 85 | 46 | 57 |
| 2 h | 82 | 55 | 69 |
| 2 h 30 | 71 | 58 | 70 |
| 3 h | 68 | 60 | 70 |
| 3 h 30 | 63 | 63 | 69 |
| 4 h | 60 | 65 | 67 |
| 4 h 30 | 59 | 67 | 65 |
| 5 h | 54 | 69 | 65 |
| 6 h | 50 | 73 | 63 |
| 8 h | 46 | 65 | 59 |

Il résulte des résultats obtenus in vitro et in vivo les constatations suivantes:

1. les essais de dissolution sont en faveur de l'effet retard des comprimés C et D par rapport aux comprimés T;
2. les essais de biodisponibilité confirment les résultats obtenus in vitro, à savoir que, par rapport aux comprimés T, les comprimés C et D présentent des caractéristiques d'un effet retard; en effet, la formulation du médicament de l'invention modifie de manière inattendue et favorable la biodisponibilité de l'acide valproïque (comprimés C et comprimés D); cette modification de la biodisponibilité permet d'éviter les effets secondaires provoqués par l'apport brusque dans le sang circulant d'acide valproïque (comprimé T); la concentration maximale se situe au temps 45 minutes, à 95 µg/ml pour le lot T, au temps 6 h, à 73 µg/ml pour le lot C et au temps 2 h 30/3 h, à 70 µg/ml pour le lot D.

Ces résultats confirment donc l'effet retard du médicament de l'invention qui permet d'écrêter la courbe de biodisponibilité et donc, d'éviter de ce fait, les effets secondaires de l'apport brusque d'acide valproïque dans le sang circulant, ainsi que les écarts très importants entre concentrations maximales et concentrations minimales, qui rendent très aléatoire le niveau de la valproïcémie à un temps quelconque lors d'administrations répétées.

D'autres essais ont été réalisés chez l'homme par administration orale d'une dose unique de 1000 mg de valproate de sodium présentée soit sous forme d'une solution buvable solution E soit sous forme de comprimés de l'invention selon la formule C à action retard. Des essais complémentaire sont été pratiqués avec les comprimés de formule C par administration de 2 comprimés en une prise par jour pendant 8 jours correspondant à une dose quotidienne de 1000 mg de valproate de sodium. Les essais ont été réalisés sur 8 sujets sains selon le schéma ci-après (les courbes obtenues sont représentées sur la figure unique en annexe):

1) Après administration unique:
les paramètres pharmacocinétiques déterminés ont été:
C max: concentration plasmatique maximale observée exprimée en $\mu g \cdot ml^{-1}$.
T max: temps nécessaire pour atteindre le C max, exprimé en heures après administration du produit
$AUC_0^\infty$: aire sous la courbe des concentrations plasmatiques d'acide valproïque en fonction du temps, exprimée en $\mu g \cdot h \cdot ml^{-1}$.

| paramètre / forme | C max | T max | $AUC_0^{\infty}$ |
|---|---|---|---|
| solution E | 90,2 ± 3,3 | 0,4 ± 0,1 | 1330 ± 117 |
| comprimé C | 50,5 ± 3,6 | 9,1 ± 0,8 | 1592 ± 129 |

L'étude de ce tableau met en évidence:

— un ralentissement de la vitesse de mise à disposition de l'acide valproïque à partir du comprimé C, objet de l'invention.

— une plus grande quantité de principe actif biodisponible après administration du médicament de l'invention expliqué par une induction enzymatique plus faible.

2) Après administration pendant 8 jours: les paramètres pharmacocinétiques déterminés ont été:

Valeurs des Cmin au Steady State

A été calculée à partir des valeurs des concentrations plasmatiques minimales obtenues avant la prise des jours $J_{19}$, $J_{20}$ et $J_{21}$ et 24h après la dernière prise de $J_{21}$.

Pour l'ensemble des 8 sujets étudiés, la moyenne est de $42{,}6 \pm 4{,}6\ \mu g \cdot ml^{-1}$.

Vitesse de passage

Les valeurs moyennes des paramètres concentration plasmatique maximale et Tmax obtenus après la dernière prise ont été respectivement de $77{,}2 \pm 5{,}8\ \mu g \cdot ml^{-1}$ et de $6{,}9 \pm 0{,}9$ h.

Aire sous la courbe dans l'intervalle entre deux prises

Les valeurs ci-après montrent que lors d'une administration réitérée, et lorsque l'état d'équilibre est atteint, l'intensité de passage du principe actif est comparable à celle qui a été calculée entre les temps 0 et infini lors d'une prise unique:

$$\overline{AUC}_{\tau n}^{\tau n+1} = 1501 \pm 126$$

$$\overline{AUC}_0^{\infty} = 1592 \pm 129$$

Cinétique d'élimination

Il a été intéressant de noter qu'à l'arrêt de l'administration répétée, la cinétique d'élimination du médicament est peu modifiée comparativement à celle qui suit la première administration.

$\beta = 0{,}036 \pm 0{,}003\ h^{-1}$ après la dernière prise

$\beta = 0{,}040 \pm 0{,}003\ h^{-1}$ après la première prise

3) A l'examen des résultats obtenus, il apparaît que:

3.1. Après administraton orale unique chez l'homme, la vitesse d'entrée de l'acide valproïque dans la circulation générale, à partir des comprimés de l'invention est nettement ralentie comparativement à une forme à libération immédiate (solution E).

A dose égale de principe actif, la quantité d'acide valproïque biodisponible est supérieure d'environ 20% lorsque l'on administre les comprimés de l'invention.

La cinétique décrite par le médicament de l'invention correspond à l'objectif recherché: écrêtement du pic plasmatique, biodisponibilité au moins égale, voire améliorée: ces critères répondent parfaitement à la définition d'une forme à libération contrôlée.

3.2. L'étude de la biodisponibilité des comprimés de l'invention a été élargie à une expérimentation d'administration chronique. Une dose équivalente à 1000 mg de valproate de sodium a été administrée en une seule prise par 24 heures jusqu'à l'obtention de l'état d'équilibre. La cinétique après la dernière prise est linéaire dans les conditions expérimentales décrites.

La forme à libération contrôlée permet de prolonger le temps de présence plasmatique dans la zone des concentrations efficaces sans atteindre des concentrations élevées responsables de phénomènes d'intolérances.

De plus, elle permet de réduire considérablement les écarts habituellement très importants entre concentrations maximales et concentrations minimales qui rendent très aléatoire le niveau de la valproïcémie à un moment quelconque lors d'administrations répétées.

Ainsi donc, les comprimés de l'invention, satisfont à l'objectif recherché et à une répercussion favorable au plan thérapeutique.

## Revendications

pour les Etats contractants BE, CH, DE, FR, GB, IT, LI, LU, NL, SE

1. Composition pharmaceutique administrable par voie orale, présentée sous forme de comprimés, caractérisée en ce qu'elle contient, à titre de principes actifs non combinés, de 25 à 35% en poids d'acide valproïque et de 75 à 65% de valproate de sodium.

2. Composition pharmaceutique selon la revendication 1, caractérisée en ce qu'elle contient de l'acide valproïque et du valproate de sodium, à raison d'une mole d'acide pour deux moles de sel.

3. Composition pharmaceutique selon l'une des revendications 1 ou 2, caractérisée en ce qu'elle contient un excipient à effet retard.

4. Composition pharmaceutique selon la revendication 3, caractérisée en ce que l'excipient contient de l'éthyl cellulose.

5. Composition pharmaceutique selon la revendication 3, caractérisée en ce que l'excipient contient de l'éthyl cellulose et de l'Eudragit®.

6. Composition pharmaceutique selon l'une quelconque des revendications 1 à 5 sous forme de dose unitaire, caractérisée en ce qu'elle contient 333 mg de valproate de sodium, 145 mg d'acide valproïque et 50 à 150 mg d'Eudragit®.

7. Composition pharmaceutique selon la revendication 6, caractérisée en ce qu'elle contient en outre de 5 à 125 mg d'éthylcellulose et de la silice.

**Revendications**

pour l'état contractant AT

1. Procédé de préparation d'une composition pharmaceutique administrable par voie orale, caractérisé en ce qu'on met sous forme de comprimés, à titre de principes actifs non combinés, de 25 à 35% en poids d'acide valproïque et de 75 à 65% de valproate de sodium.

2. Procédé selon la revendication 1, caractérisé en ce que la composition contient de l'acide valproïque et du valproate de sodium, à raison d'une mole d'acide pour deux moles de sel.

3. Procédé selon l'une des revendications 1 ou 2, caractérisé en ce que la composition contient un excipient à effet retard.

4. Procédé selon la revendication 3, caractérisé en ce que l'excipient contient de l'éthyl cellulose.

5. Procédé selon l'une des revendications 3 ou 4, caractérisé en ce que l'excipient contient de l'éthyl cellulose et de l'Eudragit®.

6. Procédé selon l'une quelconque des revendications 1 à 5 sous forme de dose unitaire, caractérisé en ce que la composition contient 333 mg de valproate de sodium, 145 mg d'acide valproïque et 50 à 150 mg d'Eudragit®.

7. Procédé selon la revendication 6, caractérisé en ce que la composition contient en outre de 5 à 125 mg d'éthylcellulose et de la silice.

**Patentansprüche**

für die Vertragsstaaten BE, CH, DE, FR, GB, IT, LI, LU, NL, SE

1. Pharmazeutische Zusammensetzung zur oralen Verabreichung in Form von Tabletten, dadurch gekennzeichnet, dass sie als aktive, nicht verbundene Bestandteile 25 bis 35 Gew.% Valproinsäure und 75 bis 65% Natriumvalproat enthält.

2. Pharmazeutische Zusammensetzung nach Anspruch 1, dadurch gekennzeichnet, dass sie Valproinsäure und Natriumvalproat im Verhältnis von einem Mol Säure je zwei Mole Salz enthält.

3. Pharmazeutische Zusammensetzung nach einem der Ansprüche 1 oder 2, dadurch gekennzeichnet, dass sie ein Bindemittel mit Retardierungswirkung enthält.

4. Pharmazeutische Zusammensetzung nach Anspruch 3, dadurch gekennzeichnet, dass das Bindemittel Ethylzellulose enthält.

5. Pharmazeutische Zusammensetzung nach einem der Ansprüche 3 oder 4, dadurch gekennzeichnet, dass das Bindemittel Ethylzellulose und Eudragit® enthält.

6. Pharmazeutische Zusammensetzung nach irgendeinem der Ansprüche 1 bis 5 als Einheitsdosis, dadurch gekennzeichnet, dass sie 333 mg Natriumvalproat, 145 mg Valproinsäure und 50 bis 150 mg Eudragit® enthält.

7. Pharmazeutische Zusammensetzung nach Anspruch 6, dadurch gekennzeichnet, dass sie ausserdem 5 bis 125 mg Ethylzellulose und Siliciumoxid enthält.

**Patentansprüche**

für den Vertragsstaat AT

1. Verfahren zur Herstellung einer oral verabreichbaren pharmazeutischen Zusammensetzung, dadurch gekennzeichnet, dass man als aktive nicht verbundene Bestandteile 25 bis 35 Gew.% Valproinsäure und 75 bis 65% Natirumvalproat in Tablettenform bringt.

2. Verfahren nach Anspruch 1, dadurch gekennzeichnet, dass die Zusammensetzung Valproinsäure und Natriumvalproat im Verhältnis von einem Mol Säure je zwei Mole Salz enthält.

3. Verfahren nach einem der Ansprüche 1 oder 2, dadurch gekennzeichnet, dass die Zusammensetzung ein Bindemittel mit Retardierungswirkung enthält.

4. Verfahren nach Anspruch 3, dadurch gekennzeichnet, dass das Bindemittel Ethylzellulose enthält.

5. Verfahren nach einem der Ansprüche 3 oder 4, dadurch gekennzeichnet, dass das Bindemittel Ethylzellulose und Eudragit® enthält.

6. Verfahren nach einem der Ansprüche 1 bis 5 in einer Einheitsdosis, dadurch gekennzeichnet, dass die Zusammensetzung 333 mg Natriumvalproat, 145 mg Valproinsäure und 50 bis 150 mg Eudragit® enthält.

7. Verfahren nach Anspruch 6, dadurch gekennzeichnet, dass die Zusammensetzung ausserdem 5 bis 125 mg Ethylzellulose und Siliciumoxid enthält.

**Claims**

for contracting states BE, CH, DE, FR, GB, IT, LI, LU, NL, SE

1. Pharmaceutical composition which can be administered by oral route presented in the form of tablets, characterized in that it contains, as non-combined active principles 25 to 35% in weight of valproic acid and 75 to 65% of sodium valproate.

2. Pharmaceutical composition as claimed in claim 1, characterized in that it contains valproic acid and sodium valproate in the ratio of one mole acid for two moles salt.

3. Pharmaceutical composition as claimed in claims 1 or 2, characterized in that it contains an excipient with delayed action.

4. Pharmaceutical composition as claimed in claim 3, characterized in that the excipient contains ethyl cellulose.

5. Pharmaceutical composition as claimed in claims 3 or 4, characterized in that the excipient contains ethyl cellulose and Eudragit®.

6. Pharmaceutical composition as claimed in any one of claims 1 to 5 in the form of unit dose, characterized in that it contains 333 mg of sodium valproate, 145 mg of valproic acid and from 50 to 150 mg of Eudragit®.

7. Pharmaceutical composition according to claim 6, characterized in that it further contains 5 to 125 mg of ethyl cellulose and silica.

**Claims**

for contracting state AT

1. Process for the preparation of a pharmaceutical composition which can be administered by oral route, characterized in that, as non-combined active principles, 25 to 35% in weight of valproic acid and 75 to 65% of sodium valproate are prepared in the form of tablets.

2. Process as claimed in claim 1, characterized in that the compositon contains valproic acid and sodium valproate in the ratio of one mole acid for two moles salt.

3. Process as claimed in claims 1 or 2, characterized in that the composition contains an excipient with delayed action.

4. Process as claimed in claim 3, characterized in that the excipient contains ethyl cellulose.

5. Process as claimed in claims 3 or 4, characterized in that the excipient contains ethyl cellulose and Eudragit®.

6. Process as claimed in any one of claims 1 to 5 in the form of unit dose, characterized in that the composition contains 333 mg of sodium valproate, 145 mg of valproic acid and from 50 to 150 mg of Eudragit®.

7. Process according to claim 6, characterized in that the composition further contains 5 to 125 mg of ethyl cellulose and silica.

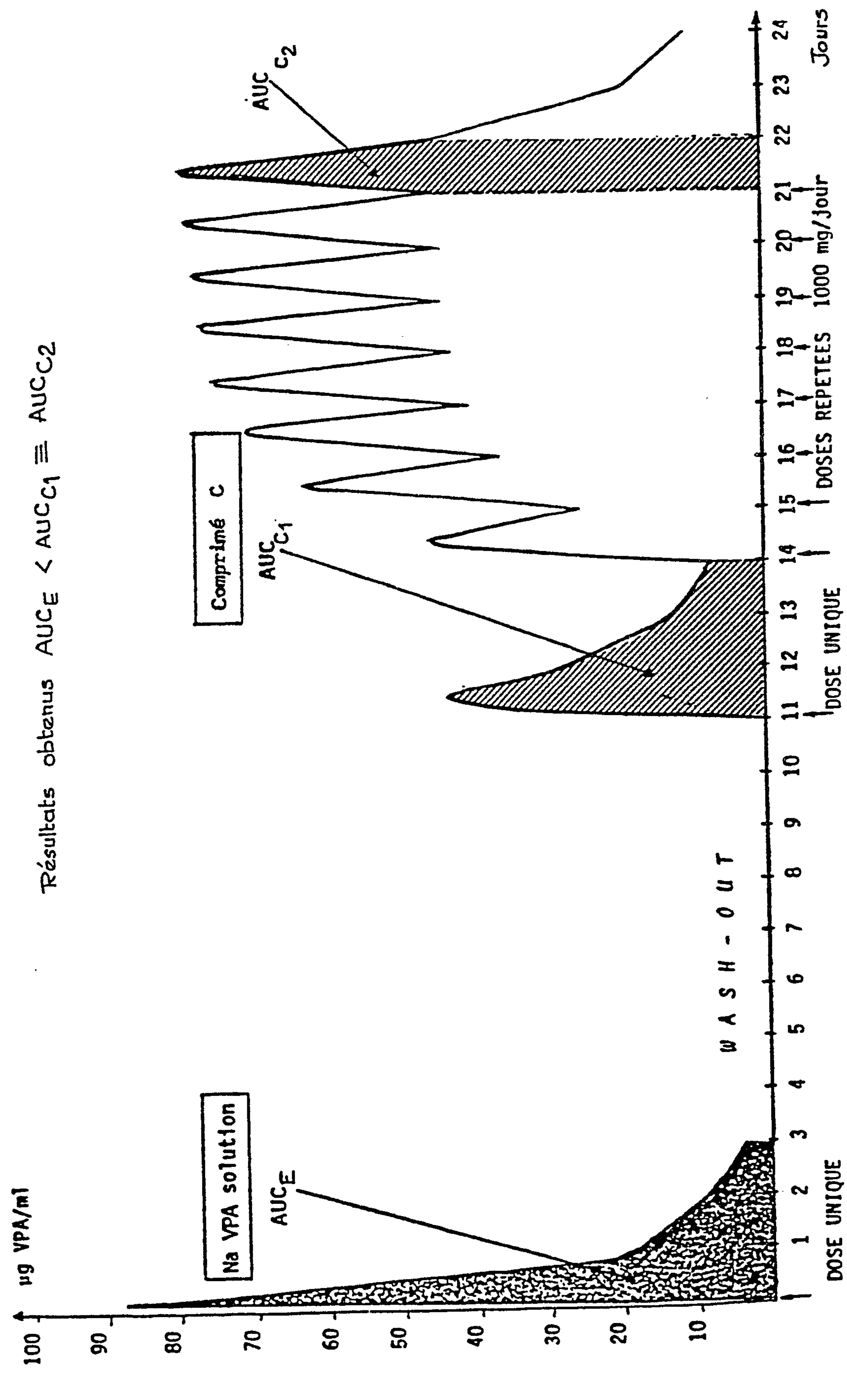
Résultats obtenus AUC_E < AUC_C1 ≡ AUC_C2
µg VPA/ml
100
90
80
70
60
50
40
30
20
10
Na VPA solution
AUC_E
Comprimé C
AUC_C1
AUC_C2
WASH - OUT
1 2 3 4 5 6 7 8 9 10 11 12 13 14 15 16 17 18 19 20 21 22 23 24
Jours
DOSE UNIQUE
1000 mg
DOSE UNIQUE
1000 mg
DOSES REPETEES 1000 mg/Jour